# EUROPEAN PATENT APPLICATION

(11) **EP 2 243 436 A2**
(43) Date of publication of application: **27.10.2010**
(21) Application number: 10250807.4
(22) Date of filing: 21.04.2010
(51) Int. Cl.: A61B 17/34, A61B 1/32, A61B 17/00, A61B 17/02, A61B 17/128, A61B 17/29, A61B 17/42, A61F 2/02, A61M 39/06

(54) **Methods and devices for identifying sealing port size**

(30) Priority: 22.04.2009 US 427964
(71) Applicant: Ethicon Endo-Surgery, Inc., Cincinnati, Ohio 45242-2839 (US)
(72) Inventor: Shelton, IV, Frederick E., Hillsboro, Ohio 45133 (US)
(74) Representative: Tunstall, Christopher Stephen

(57) **Abstract**

Methods and devices are provided for identifying surgical port size. In one embodiment, a surgical access device can include a proximal housing and a distal retractor with a plurality of sealing ports seated in the housing. The surgical access device can also include at least one identifier configured to indicate sizes of the sealing ports. The one or more identifiers can be visual and/or tactile for each of the sealing ports.

## Description

### FIELD OF THE INVENTION

The present invention relates to methods and devices for identifying sealing port size.

### BACKGROUND OF THE INVENTION

Access ports are widely used in medical procedures to gain access to anatomical cavities ranging in size from the abdomen to small blood vessels, such as veins and arteries, epidural, pleural and subarachnoid spaces, heart ventricles, and spinal and synovial cavities. The use of access ports has become more common as they provide minimally invasive techniques for establishing a portal for a number of procedures, such as those involving the abdominal cavity. Reduced postoperative recovery time, markedly decreased post-operative pain and wound infection, and improved cosmetic outcome are well established benefits of minimally invasive surgery, derived mainly from the ability of surgeons to perform an operation utilizing smaller incisions of the body cavity wall.

In many surgical procedures, it is desirable to provide one or more working channels into a body cavity through which various instruments can be passed to view, engage, and/or treat tissue to achieve a diagnostic or therapeutic effect. In laparoscopic abdominal procedures for example, the abdominal cavity is generally insufflated with CO₂ gas to a pressure of around 15 mm Hg. The abdominal wall is pierced and one or more tubular cannulas, each defining a working channel, are inserted into the abdominal cavity. A laparoscopic telescope connected to an operating room monitor can be used to visualize the operative field and can be placed through one of the working channels. Other laparoscopic instruments such as graspers, dissectors, scissors, retractors, etc. can also be placed through one or more of the working channels to facilitate various manipulations by the surgeon and/or surgical assistant(s). However, it can be difficult and time-consuming during the stress of surgery to properly differentiate between working channels, particularly in a surgical access device having multiple access ports.

Accordingly, there remains a need for methods and devices for identifying surgical access ports.

### SUMMARY OF THE INVENTION

The present invention generally provides methods and devices for identifying sealing port size. In one embodiment, a surgical access device is provided that includes a housing, and at least two sealing ports extending through the housing. Each sealing port defines a working channel extending therethrough and is configured to receive an instrument therethrough. Each working channel has a size that differs from a size of another one of the working channels, and each sealing port is associated with at least one of a visual identifier and a tactile identifier configured to indicate the size of the working channel. In some embodiments, each sealing port can be associated with a visual identifier and a tactile identifier.

The visual and tactile indicators can have a variety of configurations. For example, the visual identifier can include at least one of a color, a shape, a light, a numerical marking, an alphabetical marking, and differing vertical heights of the sealing ports, and the tactile identifier can include a textured surface.

The device can vary in any other number of ways. For example, the device can include a retractor configured to attach to the housing and having a working channel extending therethrough for forming a pathway through tissue into a body cavity. The device can also or alternatively include a tubular member extending from each sealing port and extending distally from the housing.

In another embodiment, a surgical device is provided that includes a housing having first and second sealing ports, each sealing port having a sealing element configured to form a seal around an instrument inserted through the sealing port. The first sealing port has a first diameter, and the second sealing port has a second diameter greater than the first diameter. A first identifier is configured to uniquely identify the first sealing port, and a second identifier is configured to uniquely identify the second sealing port. In some embodiments, the device can include a flexible retractor configured to couple to the housing.

The first and second identifiers can have a variety of configurations. For example, at least one of the first and second identifiers can include a visual indicator, e.g., a color, a shape, a light, an alphabetical marking, and/or a numerical marking, and/or can include a tactile feature, e.g., a textured surface. For another example, each of the first and second identifiers can have a different cross-sectional shape. For yet another example, the first and second identifiers can each include a different number of surface features, e.g., a number of surface features equal to the first and second diameters, respectively.

In another aspect, a surgical method is provided that includes positioning a surgical access device having a plurality of sealing ports within an opening formed through tissue such that each sealing port forms a working channel extending through the tissue and into a body cavity, and identifying a size of at least one of the sealing ports using at least one of a visual indicator and a tactile indicator on the surgical access device. Identifying the size of at least one of the sealing ports can include visualizing a visual indicator on the surgical access device in the form of at least one of a color, a shape, and a light, and/or manipulating a tactile indicator on the surgical access device in the form of a textured surface.

### BRIEF DESCRIPTION OF THE DRAWINGS

The invention will be more fully understood from the following detailed description taken in conjunction with the accompanying drawings, in which:

FIG. 1 is a perspective view of one embodiment of a surgical access device including a plurality of sealing ports and a plurality of visual and tactile sealing port identifiers;

FIG. 2 is a perspective view of one embodiment of a surgical access device housing seating a plurality of sealing ports and including a plurality of visual sealing port identifiers;

FIG. 3 is a perspective, partial cross-sectional view of another embodiment of a surgical access device having a plurality of sealing ports and including a plurality of visual sealing port identifiers;

FIG. 4 is a perspective view of another embodiment of a surgical access device housing seating a plurality of sealing ports and including a plurality of visual and tactile sealing port identifiers;

FIG. 5A is a perspective view of another embodiment of a surgical access device including a plurality of sealing ports and a plurality of visual and tactile sealing port identifiers;

FIG. 5B is a proximal end view of one of the sealing ports of FIG. 5A that has a circular shape;

FIG. 5C is a proximal end view of one of the sealing ports of FIG. 5A that has a square shape;

FIG. 5D is a proximal end view of one of the sealing ports of FIG. 5A that has a triangular shape;

FIG. 5E is a proximal end view of one of the sealing ports of FIG. 5A that has a star shape;

FIG. 6A is a perspective view of another embodiment of a surgical access device including a plurality of sealing ports and a plurality of visual and tactile sealing port identifiers;

FIG. 6B is a proximal end view of one of the sealing ports of FIG. 6A that has a triangular shape;

FIG. 6C is a proximal end view of one of the sealing ports of FIG. 6A that has a square shape;

FIG. 6D is a proximal end view of one of the sealing ports of FIG. 6A that has a circular shape;

FIG. 7A is a perspective view of one embodiment of a surgical access device including a plurality of sealing ports and a plurality of removable visual and tactile sealing port identifiers;

FIG. 7B is a proximal end view of one of the sealing ports of FIG. 7A that has a ring of a first color mated thereto;

FIG. 7C is a proximal end view of one of the sealing ports of FIG. 7A that has a ring of a second color mated thereto;

FIG. 7D is a proximal end view of one of the sealing ports of FIG. 7A that does not have a ring mated thereto;

FIG. 8 is a perspective view of one embodiment of a surgical access device including a plurality of sealing ports and a plurality of visual and tactile sealing port identifiers positioned in tissue, and having a surgical instrument inserted through one of the sealing ports;

FIG. 9A is a perspective view of one embodiment of a surgical access device including a sealing port and a visual and tactile sealing port identifier, and having a surgical instrument inserted through the sealing port;

FIG. 9B is a side, partial, cross-sectional view of the device of FIG. 9A positioned in tissue;

FIG. 10 is a perspective view of another embodiment of a surgical access device housing seating a plurality of sealing ports and including a plurality of visual and tactile sealing port identifiers;

FIG. 11 is a perspective view of yet another embodiment of a surgical access device housing seating a plurality of sealing ports and including a plurality of visual and tactile sealing port identifiers;

FIG. 12 is a perspective view of still another embodiment of a surgical access device housing seating a plurality of sealing ports and including a plurality of visual and tactile sealing port identifiers;

FIG. 13 is a perspective view of another embodiment of a surgical access device housing seating a plurality of sealing ports and including a plurality of visual and tactile sealing port identifiers;

FIG. 14 is a perspective view of yet another embodiment of a surgical access device housing seating a plurality of sealing ports and including a plurality of visual and tactile sealing port identifiers;

FIG. 15 is a perspective, partial view of one embodiment of a surgical access device housing seating a sealing port and including a visual and tactile sealing port identifier;

FIG. 16 is a side, partial cross-sectional view of another embodiment of a surgical access device including a plurality of sealing ports and a plurality of visual and tactile sealing port identifiers;

FIG. 17 is a perspective, partial view of another embodiment of a surgical access device including a plurality of sealing ports and a plurality of visual and tactile sealing port identifiers positioned in tissue; and

FIG. 18 is a side, partial cross-sectional view of another embodiment of a surgical access device including a plurality of sealing ports and a plurality of visual sealing port identifiers positioned in tissue, and having a surgical instrument inserted through one of the sealing ports.

### DETAILED DESCRIPTION OF THE INVENTION

Certain exemplary embodiments will now be described to provide an overall understanding of the principles of the structure, function, manufacture, and use of the devices and methods disclosed herein. One or more examples of these embodiments are illustrated in the accompanying drawings. Those skilled in the art will understand that the devices and methods specifically described herein and illustrated in the accompanying drawings are non-limiting exemplary embodiments and that the scope of the present invention is defined solely by the claims. The features illustrated or described in connection with one exemplary embodiment may be combined with the features of other embodiments. Such modifications and variations are intended to be included within the scope of the present invention.

Various exemplary methods and devices are provided for identifying sealing port size. In one embodiment, a surgical access device can include a proximal housing and a distal retractor with a plurality of sealing ports located in the housing. It can be difficult to uniquely identify the size of each of the sealing ports, particularly when at least two sealing ports of the surgical access device define working channels of a similar, small size, e.g., differing by equal to or less than about 2 mm. Hence, it can be difficult to quickly and easily determine which one of a plurality of available surgical access devices to use in a particular context in a particular surgical procedure and/or to quickly and easily determine which one of a plurality of sealing ports to use once a surgical access device is positioned in tissue. The surgical access device can thus also include at least one identifier configured to indicate sizes of the sealing ports. In an exemplary embodiment, the device can include at least one visual and/or tactile identifier for each of the sealing ports, thereby facilitating fast, easy identification of the sealing ports without requiring measurement of the sealing ports.

The various surgical access devices described herein can generally be configured to allow one or more surgical instruments to be inserted therethrough through one or more independent sealing ports or access ports formed in a proximal housing, hereinafter generally referred to as a housing, of the device and into a body cavity. The sealing ports can each define working channels extending through the proximal housing and aligned with a distal retractor. The distal retractor, hereinafter generally referred to as a retractor, can be configured as a wound protector, or other member for forming a pathway through tissue. The retractor can extend from the proximal housing of the device, and it can be configured to be positioned within an opening in a patient's body, such as the umbilicus. Any and all of the surgical access devices described herein can also include various other features, such as one or more ventilation ports to allow evacuation of smoke during procedures that utilize cautery, and/or one or more insufflation ports through which the surgeon can insufflate the abdomen to cause pneumoperitenium, as described by way of non-limiting example in U.S. Patent Application No. 2006/0247673 entitled "Multi-port Laparoscopic Access Device" filed November 2, 2006, which is hereby incorporated by reference in its entirety. The insufflation port can be located anywhere on the device, can have any size, and can accept a leur lock or a needle, as will be appreciated by those skilled in the art.

Any and all embodiments of a surgical access device can also include one or more safety shields positioned through, in, and around any of the components and/or tissue to protect the components against puncture or tear by surgical instruments being inserted through the device. Exemplary embodiments of safety shields are described in more detail in U.S. Patent Publication No. 2006/0247673 entitled "Multi-port Laparoscopic Access Device" filed November 2, 2006, U.S. Patent Application No. 12/399,625 entitled "Methods and Devices for Providing Access to a Body Cavity" filed on March 6, 2009, U.S. Patent Application No. 12/399,482 entitled "Methods and Devices for Providing Access to a Body Cavity" filed on March 6, 2009, and U.S. Patent Application No. 12/242,765 entitled "Surgical Access Device" filed on September 30, 2008, which are hereby incorporated by reference in their entireties.

In any and all of the surgical access device embodiments disclosed herein, an engagement and/or release mechanism can be included to allow certain components of the surgical access device to be removable as needed, such as removable coupling of a housing and a retractor. Any engagement and release mechanism known in the art, e.g., a snap-lock mechanism, corresponding threads, etc., can be used to releasably mate components of the device.
Exemplary embodiments of an engagement and release mechanisms are described in more detail in previously mentioned U.S. Patent Application No. 12/242,765 entitled "Surgical Access Device" filed on September 30, 2008, U.S. Patent Application No. 12/399,625 entitled "Methods and Devices for Providing Access to a Body Cavity" filed on March 6, 2009, and U.S. Patent Application No. 12/399,482 entitled "Methods and Devices for Providing Access to a Body Cavity" filed on March 6, 2009 and in U.S. Patent No. 7,371,227 entitled "Trocar Seal Assembly," issued May 13, 2008 and U.S. Patent No. 5,628,732 entitled "Trocar With Improved Universal Seal," issued May 13, 2007, which are hereby incorporated by reference in their entireties.

In use, as further discussed below, the surgical access devices disclosed herein can be used to provide access to a patient's body cavity. The device's retractor can be positionable within an opening in a patient's body such that a distal portion of the retractor extends into a patient's body cavity and a proximal portion configured to couple to the device's housing is positioned adjacent to the patient's skin on an exterior of the patient's body. A lumen in the retractor can form a pathway through the opening in a patient's body so that surgical instruments can be inserted from outside the body to an interior body cavity. The elasticity of the skin of the patient can assist in the retention of the retractor in the body opening or incision made in the body. The retractor can be placed in any opening within a patient's body, whether a natural orifice or an opening made by an incision. As a non-limiting example, the retractor can be placed through the umbilicus. In one embodiment, the retractor can be substantially flexible so that it can easily be maneuvered into and within tissue as needed. In other embodiments, the retractor can be substantially rigid or substantially semi-rigid. The retractor can be formed of any suitable material known in the art, e.g., silicone, urethane, thermoplastic elastomer, and rubber.

Typically, during surgical procedures in a body cavity, such as the abdomen, insufflation is provided through the surgical access device to expand the body cavity to facilitate the surgical procedure. Thus, in order to maintain insufflation within the body cavity, most surgical access devices include at least one seal disposed therein to prevent air and/or gas from escaping when surgical instruments are inserted therethrough. Various sealing elements are known in the art, but typically the surgical access device can include at least one instrument seal that forms a seal around an instrument disposed therethrough, but otherwise does not form a seal when no instrument is disposed therethrough; at least one channel seal or zero-closure seal that seals the working channel created by the sealing port when no instrument is disposed therethrough; or a combination instrument seal and channel seal that is effective to both form a seal around an instrument disposed therethrough and to form a seal in the working channel when no instrument is disposed therethrough. A person skilled in the art will appreciate that various seals known in the art can be used including, e.g., duckbill seals, cone seals, flapper valves, gel seals, diaphragm seals, lip seals, iris seals, etc. A person skilled in the art will also appreciate that any combination of seals can be included in any of the embodiments described herein, whether or not the seal combinations are specifically discussed in the corresponding description of a particular embodiment. Exemplary embodiments of various seal protectors are described in more detail in U.S. Patent No. 5,342,315 entitled "Trocar Seal/Protector Assemblies," issued August 30, 1994 and U.S. Patent No. 7,163,525 entitled "Duckbill Seal Protector," issued January 16, 2007, which are hereby incorporated by reference in their entireties.

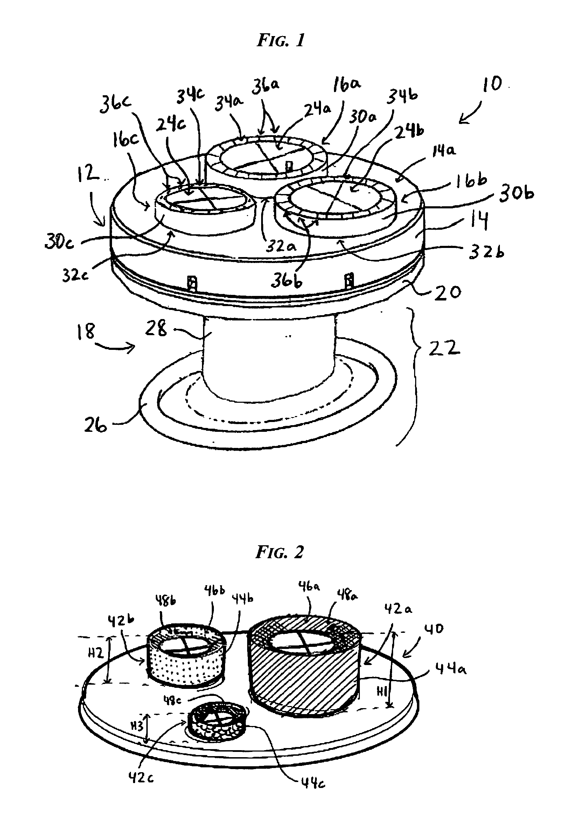

In an exemplary embodiment, shown in FIG. 1, a surgical access device 10 is provided having a housing 12 configured to have one or more surgical instruments inserted therethrough. Although the housing 12 can have any configuration, in this illustrated embodiment, the housing 12 includes a seal base 14 configured to support at least one sealing or access port (not shown) and configured to form a seat and seal between the base 14 and a distal portion of the device 10, e.g., a retractor 18. The housing 12 can be fixedly or removably coupled to the retractor 18 configured to distally extend from the housing 12 and to provide a pathway through tissue into a body cavity. In this embodiment, the retractor 18 includes a proximal retractor portion or proximal retractor base 20 coupled to a distal retractor portion 22.

As noted above, the retractor 18 can extend distally from the housing 12, and it can be configured to be positioned in an opening formed in tissue. The retractor 18 can, as shown in this exemplary embodiment, include a substantially flexible distal portion 22 having a proximal flange (not shown) and a distal flange 26 with an inner elongate portion 28 extending therebetween. A retractor retaining band (not shown), e.g., an o-ring, can be positioned between the proximal retractor base 20 and the flexible distal portion 22 to help form a secure seal therebetween. The inner elongate portion 28 can have a diameter less than a diameter of the proximal flange and the distal flange 26, which can have the same diameter or different diameters from one another, and can be configured to be positioned within tissue. The proximal flange can be configured to be seated within the proximal retractor base 20 as illustrated in this embodiment, or the proximal retractor base 20 can be configured to be seated within the proximal flange. The proximal retractor base 20 can optionally be attached to the proximal flange using an adhesive, sealant, complementary threads, or any other attachment mechanism, as will be appreciated by a person skilled in the art. A proximal o-ring (not shown) can optionally be positioned within the proximal flange to help provide structural support to the retractor 18 if the proximal flange is seated within the proximal retractor base 20. A distal o-ring (not shown) can optionally be positioned within the distal flange 26 to provide structural support to the retractor 18 within a patient's body. The proximal and distal o-rings can be substantially flexible or substantially rigid as needed, same or different from one another, for use in a particular application.

As shown in this embodiment, the housing 12 can be removably coupled via snap-fit to the retractor 18, which as illustrated in this embodiment can be flexible. The housing 12 can be in a fixed position relative to the retractor 18 as shown in this embodiment, or the housing 12 can be movable relative to the retractor 18. Exemplary embodiments of various housings are described in more detail in previously mentioned U.S. Patent Publication No. 2006/0247673 entitled "Multi-port Laparoscopic Access Device" filed November 2, 2006, U.S. Patent Application No. 12/399,625 entitled "Methods and Devices for Providing Access to a Body Cavity" filed on March 6, 2009, U.S. Patent Application No. 12/399,482 entitled "Methods and Devices for Providing Access to a Body Cavity" filed on March 6, 2009, and U.S. Patent Application No. 12/242,765 entitled "Surgical Access Device" filed on September 30, 2008, and in U.S. Patent Application No. 12/399,547 entitled "Surgical Access Devices And Methods Providing Seal Movement In Predefined Paths" filed on March 6, 2009, which is hereby incorporated by reference in its entirety.

While any number of sealing ports can be formed in the seal base 14, in this illustrated embodiment, the seal base 14 includes first, second, and third sealing port openings (not shown) formed therein that extend through the seal base 14 in which first, second, and third sealing ports 16a, 16b, 16c can be seated. In general, the sealing ports 16a, 16b, 16c can define a working channel (not shown) extending therethrough and be configured to receive an instrument therethrough. Each of the sealing ports 16a, 16b, 16c can include a port housing 30a, 30b, 30c, which can be seated directly or indirectly in one of the port openings in the seal base 14, and a sealing element 24a, 24b, 24c, which can be positioned within an associated port housing 30a, 30b, 30c. The port housings 30a, 30b, 30c can each have any shape, height, or angular configuration, but in the embodiment shown in FIG. 1, the port housings 30a, 30b, 30c can each have a cylindrical shape. First, second, and third distal surfaces 32a, 32b, 32c of the respective port housings 30a, 30b, 30c can be substantially flat such that they can be coplanar with a proximal surface 14a of the seal base 14, as shown. First, second, and third proximal surfaces 34a, 34b, 34c of the respective port housings 30a, 30b, 30c can likewise be flat, or any one or more can extend at an angle with respect to the proximal surface 14a of the seal base 14, such as described in more detail in previously mentioned U.S. Patent Application No. 12/242,765 entitled "Surgical Access Device" filed on September 30, 2008. The proximal surfaces 34a, 34b, 34c can also include first, second, and third surface features 36a, 36b, 36c, which are discussed further below. A sealing element can include at least one instrument seal and/or at least one channel seal, and can generally be configured to contact an instrument inserted through the sealing element's associated sealing port. Exemplary embodiments of various sealing ports are described in more detail in previously mentioned U.S. Patent Publication No. 2006/0247673 entitled "Multi-port Laparoscopic Access Device" filed November 2, 2006, U.S. Patent Application No. 12/399,625 entitled "Methods and Devices for Providing Access to a Body Cavity" filed on March 6, 2009, U.S. Patent Application No. 12/399,482 entitled "Methods and Devices for Providing Access to a Body Cavity" filed on March 6, 2009, and U.S. Patent Application No. 12/242,765 entitled "Surgical Access Device" filed on September 30, 2008.

The sealing ports 16a, 16b, 16c can, as in this illustrated embodiment, each have a central axis that extends substantially perpendicular to the proximal surface 14a of the seal base 14, and the sealing ports 16a, 16b, 16c can each be in a fixed position relative to the housing 12, but any one or more of the sealing ports can be angled relative to the seal base 14 and/or rotatable or otherwise movable relative to the seal base 14 and/or other portion(s) of the housing 12. Additionally or alternatively, any one or more of the sealing ports 16a, 16b, 16c can be configured to be movable relative to any one or more portions of the retractor 18 and/or any others of the sealing ports 16a, 16b, 16c. The sealing ports 16a, 16b, 16c can be attached or mated to the seal base 14 using any attachment or mating mechanism known in the art, but in the illustrated embodiment the sealing ports 16a, 16b, 16c can each mate with the seal base 14 through an interference fit.

The sealing ports 16a, 16b, 16c can each have any size, e.g., working channel diameter configured to allow passage of a surgical instrument having a diameter equal to or less than the working channel diameter. At least two of the sealing ports 16a, 16b, 16c can have different sizes. As shown in this embodiment, the first and second sealing ports 16a, 16b can each have a first diameter D1 configured to allow passage therethrough of a surgical instrument having a diameter equal to or less than the first diameter D1, while the third sealing port 16c can have a second diameter D2 smaller than the first diameter D1 and configured to allow passage therethrough of a surgical instrument having a diameter equal to or less than the second diameter D2. The first diameter D1 can be about 5 mm, and the second diameter D2 can be about 3 mm, although the diameters D1, D2 can have any size, e.g., 10 mm, 12 mm, 7 mm, 3 mm, 5 mm, etc.

The device 10 can include one or more identifiers configured to indicate sizes of the sealing ports 16a, 16b, 16b. In an exemplary embodiment the one or more identifiers can be located on the sealing ports 16a, 16b, 16c, e.g., on the port housings 30a, 30b, 30c and/or on the sealing elements 24a, 24b, 24c, although the one or more identifiers can additionally or alternatively be located anywhere on the device 10, e.g., on the proximal base surface 14a. The identifiers can be integral with the device 10 or can be removably coupled to the device 10. The device 10 can include any number of identifiers, although in an exemplary embodiment a quantity of the identifiers can be equal to or greater than a number of the sealing ports 16a, 16b, 16c to allow each of the sealing ports 16a, 16b, 16c to be associated with and uniquely identified by at least one identifier. Each of the sealing ports 16a, 16b, 16c can have a different identifier from the others of the sealing ports 16a, 16b, 16c, which can help uniquely identify each of the sealing ports 16a, 16b, 16c. In embodiments of surgical access devices having at least one sealing port with the same size as any one or more of the device's other sealing ports, such as the first and second sealing ports 16a, 16b in this illustrated embodiment that each have the same diameter D1, the same-sized sealing ports can have identical identifiers as one another. Alternatively or in addition, same-sized sealing ports can each have at least one identical identifier and each have at least one different identifier which can allow identification of the same-sized sealing ports as having the same size while providing additional visual and/or tactile identification information related to another feature of the same-sized sealing ports, such as their angles with respect to the proximal surface 14a of the seal base 14, the longitudinal length of their sealing elements, etc.

The one or more identifiers can have a variety of configurations, and can be visual, e.g., be seen, and/or tactile, e.g., be felt. Exemplary embodiments of a visual identifier include a color, a shape, a light, a numerical and/or alphabetical marking, a pattern such as stripes, dots, a checkerboard, zig-zags, etc., a vertical sealing port height, and a quantity of surface features, although any visual marking can be used. Exemplary embodiments of a tactile identifier include a protrusion, a depression, a textured surface, and a shape, although any tactile feature can be used. In some embodiments of a surgical access device, one or more of the device's sealing ports having the same size can lack an identifier, which can itself be an identifier that uniquely identifies a size of the one or more same-sized sealing ports in relation to other, differently-sized sealing ports of the device that can each be associated with at least one visual and/or tactile identifier. A key or legend can be printed on or otherwise provided with the surgical access device 10 to correlate any one or more visual and/or tactile identifiers with the sealing port sizes they identify, e.g., indicating that the color red corresponds to a 3 mm sealing port, that raised tactile ridges correspond to a 12 mm sealing port, etc.

In the illustrated embodiment, the first, second, and third surface features 36a, 36b, 36c on the proximal surfaces 34a, 34b, 34c of the sealing ports 16a, 16b, 16b can be configured as identifiers integrally formed with the sealing ports 16a, 16b, 16c to help identify respective sealing port sizes of the sealing ports 16a, 16b, 16c. As shown, the first, second, and third surface features 36a, 36b, 36c can each include a pattern printed, embossed, or otherwise applied to the respective proximal surfaces 34a, 34b, 34c, with the first and second surface features 36a, 36b of the same-sized sealing ports 16a, 16b being identical, e.g., at least one ridge depressed in the first and second proximal surfaces 34a, 34b, and the third surface feature 36c for the third sealing port 16c being different, e.g., at least one raised bump protruding from the third proximal surface 34c, and hence distinguishable from the first and second surface features 36a, 36b. The surface features 36a, 36b, 36c can thus be configured as visual and tactile identifiers that can be visually identified and/or tactilely identified through their different patterns. Each of the port housings 30a, 30b, 30c in this illustrated embodiments extend a same vertical height above or proximal to the proximal surface 14a of the seal base 14, but as mentioned above and as discussed further below, the vertical heights can vary between one or more of the sealing ports 16a, 16b, 16c and be a port size identifier in addition or as an alternative to the surface features 34a, 34b, 34c.

FIG. 2 illustrates another embodiment of a housing 40 configured to seat first, second, and third sealing ports 42a, 42b, 42c and that includes a plurality of visual identifiers. The housing 40, as well as other embodiments of housings described herein, can be configured and used similar to the housing 12 discussed above as part of a surgical access device that can be configured to be releasably or fixedly mated to a retractor (not shown), and the sealing ports 42a, 42b, 42c, as well as other embodiments of sealing ports described herein, can be configured and used to the sealing ports 16a, 16b, 16c discussed above. In this embodiment, each of the sealing ports 42a, 42b, 42c can have a different size and can be associated with first, second, and third visual identifiers. The first visual identifier can be in the form of a color, e.g., red for the first sealing port 42a, green for the second sealing port 42b, and blue for the third sealing port 42c, although these or any color can be used for any of the identifiers. The colors of the first, second, and third sealing ports 42a, 42b, 42c are solid in this embodiment, but the colors can have any same or different pattern on any of the sealing ports 42a, 42b, 42c. Further, port housings 44a, 44b, 44c of the sealing ports 42a, 42b, 42c include the color identifiers, but as mentioned above, the visual identifiers can be located anywhere in addition or in the alternative.

The second visual identifier can be in the form of varied sealing port heights. As shown in this embodiment, the first, second, and third sealing ports 42a, 42b, 42c can have different heights H1, H2, H3, respectively. The heights H1, H2, H3 can visually indicate relative size with the largest sealing port having the largest height and with the heights decreasing for each subsequent smaller sealing port such that the smallest sealing port can have the smallest height, although the heights of the sealing ports can vary in any way.

The third visual identifier can be in the form of a printed alpha-numeric marking 48a, 48b, 48c on one or more of the sealing ports 42a, 42b, 42c. As shown, proximal surfaces 46a, 46b, 46c of the sealing ports 42a, 42b, 42c can each include a printed or otherwise applied respective alpha-numeric marking 48a, 48b, 48c indicating a port size, e.g., 10 mm for the first sealing port 42a, 5 mm for the second sealing port 42b, and 3 mm for the third sealing port 42c.

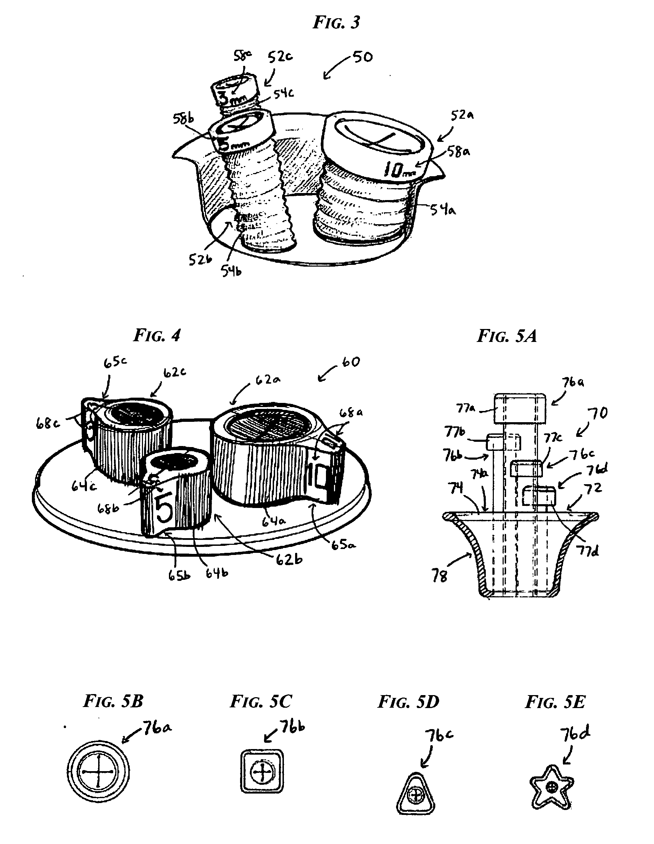

FIG. 3 illustrates another embodiment of a housing 50 configured to seat a plurality of sealing ports 52a, 52b, 52c and including a plurality of visual identifiers. In this embodiment, the visual identifiers include a first visual identifier in the form of differing vertical heights of the sealing ports 52a, 52b, 52c, and a second visual identifier in the form of alpha-numeric markings 58a, 58b, 58c printed on or otherwise applied to sidewalls of the sealing ports' respective port housings 54a, 54b, 54c indicating respective sizes of the sealing ports 52a, 52b, 52c. FIG. 3 also illustrates an embodiment of flexible sealing ports 52a, 52b, 52c having flexible port housings 54a, 54b, 54c.

FIG. 4 illustrates another embodiment of a housing 60 configured to seat a plurality of sealing ports 62a, 62b, 62c and including visual identifiers in the form of numerical markings 68a, 68b, 68c. The numerical markings 68a, 68b, 68c can be applied in any way to the sealing ports 62a, 62b, 62c, respectively. If the markings 68a, 68b, 68c are embossed as depressions or raised as protrusions, the markings 68a, 68b, 68c can be configured as tactile features that can allow the sealing ports 62a, 62b, 62c to be identified by touch. In this embodiment, the sealing ports 62a, 62b, 62c each include respective port housings 64a, 64b, 64c in the form of dials having a generally cylindrical shape with a protrusion 65a, 65b, 65c extending radially outward therefrom. The numerical markings 68a, 68b, 68c can be located anywhere on the surgical access device including the housing 60, such as shown with one numerical marking on a proximal surface of each dial protrusion 65a, 65b, 65c and another numerical marking on a sidewall of each dial protrusion 65a, 65b, 65c.

FIGS. 5A-5E illustrate yet another embodiment of a surgical access device 70 that includes a retractor 78 and a housing 72 configured to couple to the retractor 78 and having a seal base 74 configured to seat a plurality of sealing ports 76a, 76b, 76c, 76d. The retractor 78, as well as other embodiments of retractors described herein, can be configured and used similar to the retractor 18 discussed above. In this embodiment, the device 70 includes a visual indicator in the form of differing vertical heights of the sealing ports 76a, 76b, 76c, 76d above or proximal to a proximal surface 74a of the seal base 74, with decreasing heights with the largest sealing port 76a having the largest height and the smallest sealing port 76d having the smallest height. The device 70 also includes visual and tactile indicators in the form of different shapes of each of the sealing ports 76a, 76b, 76c, 76d. Proximal portions 77a, 77b, 77c, 77d of each of the sealing ports 76a, 76b, 76c, 76d can each have different cross-sectional shapes, e.g., a circle for the first sealing port 76a as shown in FIG. 5B, a square for the second sealing port 76b as shown in FIG. 5C, a triangle for the third sealing port 76c as shown in FIG. 5D, and a star for the fourth sealing port 76d as shown in FIG. 5E, although any other shapes can be used. In this way, the different sized sealing ports 76a, 76b, 76c, 76d can be easily, uniquely identified at least from above, such as when the device 70 is positioned in tissue with the seal base 74 positioned outside the patient's body. Although only the proximal portions 77a, 77b, 77c, 77d of the sealing ports 76a, 76b, 76c, 76d have different cross-sectional shapes from each other, a person skilled in the art will appreciate that any or all portions of the sealing ports 76a, 76b, 76c, 76d can have different cross-sectional shapes.

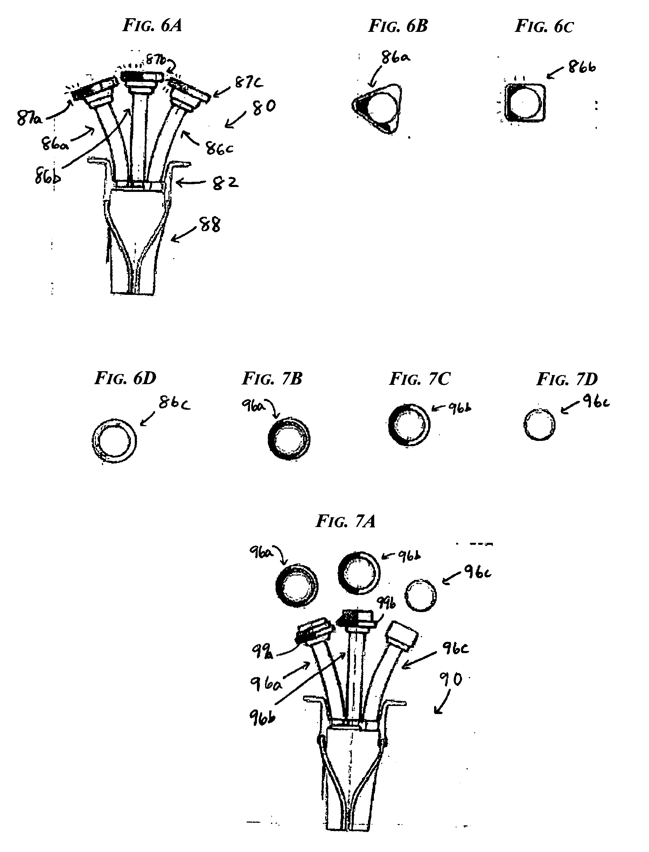

FIGS. 6A-6D illustrate another embodiment of a surgical access device 80 that includes a retractor 88 and a housing 82 configured to couple to the retractor 88 and to seat a plurality of sealing ports 86a, 86b, 86c. In this embodiment, the device 80 includes a visual and tactile indicator in the form of different shapes of each of the sealing ports 86a, 86b, 86c, similar to the different shapes of the sealing ports 76a, 76b, 76c, 76d of FIGS. 5A-5E discussed above. The device 80 also includes a visual indicator in the form of different colored lights for each of the differently sized sealing ports 86a, 86b, 86c, which can be particularly effective for identification of sealing ports in a dark operating room. The lights can have any color, and the lights can be solid and/or blinking at any frequency. The lights can be disposed in any portion of the device 80, such as in approximately one half of proximal portions 87a, 87b, 87c of the sealing ports 86a, 86b, 86c, which can help distance the lights from the surgical site and help reduce interference of the lights with a surgical procedure. The device 80 can include an actuator (not shown), e.g., an on/off button, switch, a removable insulator configured to prevent electrical conductivity until removed from the device 80, etc., configured to turn the lights on when the device 80 is in use and to turn the lights off when the device 80 is not in use.

As mentioned above, in some embodiments a surgical access device can include one or more removable visual and/or tactile identifiers. FIGS. 7A-7D illustrate an exemplary embodiment of a surgical access device 90 having one or more removable visual and/or tactile identifiers that includes a retractor 98 and a housing 92 configured to couple to the retractor 98 and to seat a plurality of sealing ports 96a, 96b, 96c. In this embodiment, the device 90 includes removable, differently colored rings 99a, 99b configured to be mated to at least one of the sealing ports 96a, 96b, 96c. Each of the sealing ports 96a, 96b, 96c can each receive any number of the rings 99a, 99b, such as shown with the first and second sealing ports 96a, 96b each receiving one of the rings 99a, 99b and the third sealing port 96c not receiving a ring. The rings 99a, 99b, which can include any colors, can be sized such that they can be disposed over proximal ends of their respective sealing ports 96a, 96b and slide distally any distance, e.g., until attaching to the sealing ports 96a, 96b with a snap-fit.

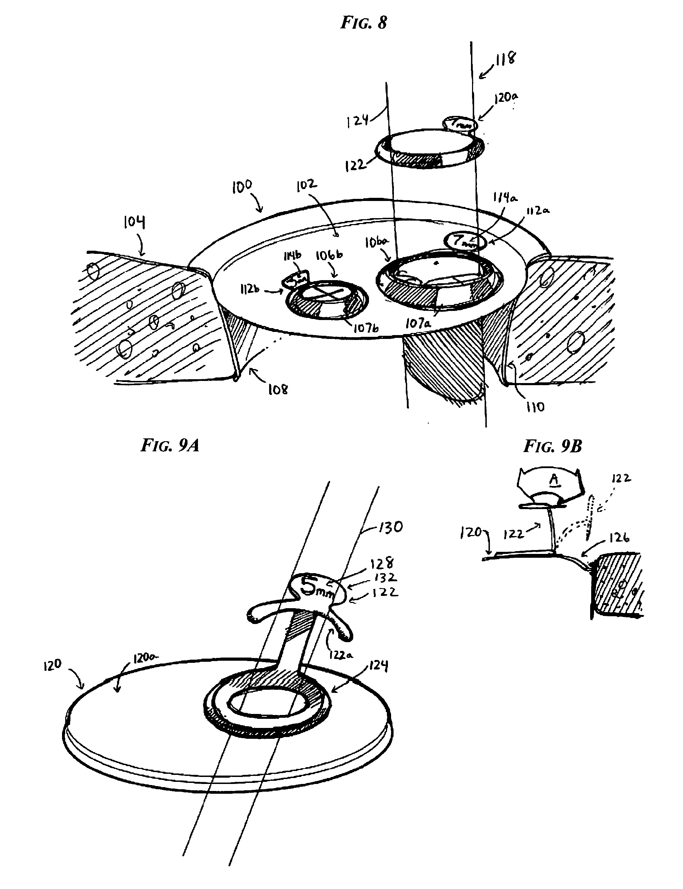

FIG. 8 illustrates another embodiment of a surgical access device 100 that includes a retractor 108 and a housing 102 configured to couple to the retractor 108 and to seat first and second sealing ports 106a, 106b. FIG. 8 also shows the device 100 with an inner elongate portion 110 of the retractor 108 positioned in tissue 104 with a proximal portion of the device 100, e.g., at least a portion of the housing 102, positioned outside the patient's body. In this embodiment, the device 100 can include first and second tags 112a, 112b in a proximal portion of the first and second sealing ports 106a, 106b, respectively. The first and second tags 112a, 112b can be configured as visual and tactile identifiers by having different shapes, e.g., triangular and circular, and/or by having alpha-numeric markings 114 printed or otherwise applied to the tags 112a, 112b to indicate sizes of the sealing ports 106a, 106b, e.g., 3 mm and 7 mm. The tags 112a, 112b can be configured to engage a corresponding tag on a surgical instrument by extending radially outward from the sealing ports 106a, 106b, e.g., from port housings 107a, 107b of the sealing ports 106a, 106b, from the housing 102 adjacent the sealing ports 106a, 106b, etc. In this way, a surgical instrument 118 can be inserted through the one of the sealing ports 106b (or multiple ones of the sealing ports) having a tag 120 radially extending outward from the instrument 118 with a shape and alpha-numeric marking matching the sealing port's tag 112a, which in this embodiment includes a circular shape and a 7 mm alpha-numeric marking. The instrument's tag 120 can be fixedly or removably coupled to the instrument 118. In the illustrated embodiment, the tag 120 extends radially outward from a ring 122 disposed around an elongate shaft 124 of the surgical instrument 118. The sealing port tags 112a, 112b and the instrument tag 118 can optionally be angled in a proximal direction, as shown, which can help make the alpha-numeric markings on the tags 112a, 112b, 118 easier to see.

FIGS. 9A and 9B illustrate another embodiment of a housing 120 that includes an identifier in the form of a tag 122. In this embodiment, the tag 122 can extend proximally from the sealing port 124, or from other location such as from a proximal surface 120a of the housing 120, and be configured as an identifier with a printed or otherwise applied alpha-numeric marking 128 indicating a size of the sealing port 124, e.g., 5 mm. In an exemplary embodiment the tag 122 can be flexible, as shown by the directional arrow A in FIG. 9B, although the tag 122 can be rigid or substantially semi-rigid. Being flexible, the tag 122 can flex to provide better angular positioning of a surgical instrument inserted through the sealing port 124.

The tag 122 can have any size and shape. In an exemplary embodiment, the tag 122 can have a wishbone-like shape and include a tab 132 with the alpha-numeric marking 128 and a contoured surface 122a configured to engage an elongate shaft 130 of a surgical instrument inserted through the sealing port 124. The contoured surface 122a can be configured as an identifier of the sealing port's size by having a degree of curvature equal to a degree of curvature of the sealing port 124, e.g., a degree of curvature corresponding to a 5 mm working channel. In this way, engaging the elongate shaft 130 of the instrument with the contoured surface 122a can help visually and/or tactilely indicate if the surgical instrument is too large or too small to be properly inserted through the sealing port 124. The contoured surface 122a can have a variety of shapes, e.g., c-shaped (as shown), circular, etc., configured to correspond to an outer surface shape of surgical instruments configured to be inserted into a body through a surgical access device.

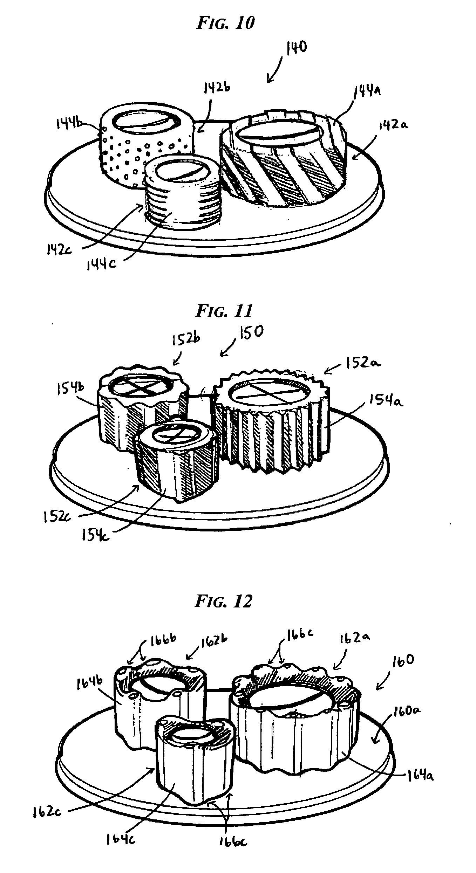

FIG. 10 illustrates another embodiment of a housing 140 configured to seat first, second, and third sealing ports 142a, 142b, 142c and including a plurality of identifiers. In this embodiment, each of the differently sized sealing ports 142a, 142b, 142c can include a port housing 144a, 144b, 144c having a different texture that can be visually and/or tactilely identified to uniquely identify each of the sealing ports 142a, 142b, 142c. The first sealing port 142a can include a texture of alternating raised and depressed diagonal ridges, the second sealing ports 142b can include a texture of raised bumps, and the third sealing port 142c can include a series of horizontal raised rings, although as mentioned above any textured surface can be used to indicate any sealing port size.

The port housings 144a, 144b, 144c can each have a substantially circular cross-sectional shape as shown in FIG. 10. In another embodiment of a housing 150, illustrated in FIG. 11, port housings 154a, 154b, 154c of sealing ports 152a, 152b, 152c seated in the housing 150 can have non-circular cross-sectional shapes configured as visual and/or tactile identifiers. The port housings 154a, 154b, 154c of the differently-sized sealing ports 152a, 152b, 152c can each have a different cross-sectional shape such that the port housings 154a, 154b, 154c can be visualized to uniquely identify each of the sealing ports 152a, 152b, 152c. The different cross-sectional shapes of the port housings 154a, 154b, 154c can provide different textures to each of the sealing ports 152a, 152b, 152c such that they can additionally or alternatively be touched to tactilely identify the sealing ports 152a, 152b, 152c.

FIG. 12 illustrates another embodiment of a housing 160 configured to seat first, second, and third sealing ports 162a, 162b, 162c having non-circular cross-sectional shapes configured as visual and/or tactile identifiers. As mentioned above, port housings 164a, 164b, 164c of the sealing ports 162a, 162b, 162c can have any cross-sectional shape, but in an exemplary embodiment, the port housings 164a, 164b, 164c can each include a different number of surface features 166a, 166b, 166c, which can help differentiate between the sealing ports 162a, 162b, 162c. The port housings 164a, 164b, 164c can each include any number of surface features 166a, 166b, 166c, such as a number of surface features 166a, 166b, 166c equal to a size of the sealing ports 162a, 162b, 162c, e.g., eleven surface features 166a for the 11 mm diameter sealing port 162a, five surface features 166b for the 5 mm diameter sealing port 162b, three surface features 166c for the 3 mm diameter sealing port 162c. The surface features 166a, 166b, 166c can thus be configured as identifiers that uniquely identify sizes of the sealing ports 162a, 162b, 162c.

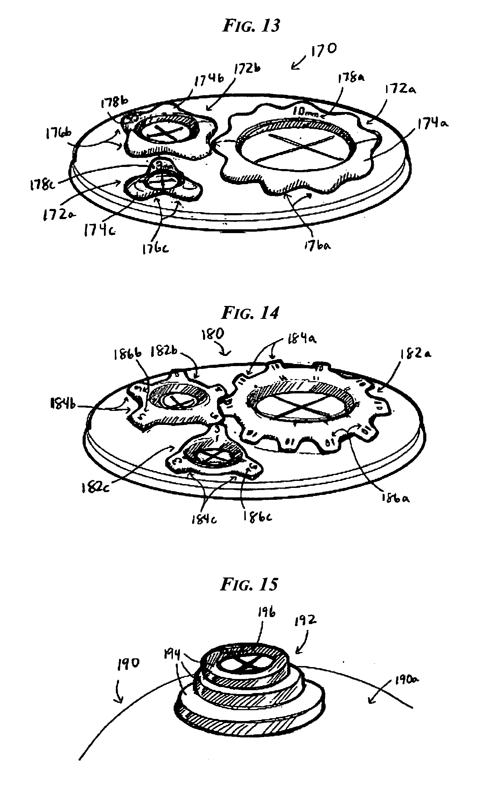

The port housings 164a, 164b, 164c can extend a distance proximally above a proximal surface 160a of the housing 160 as shown in FIG. 12. Alternatively or in addition, port housings can be flush with or recessed in the proximal housing surface 160a. As shown in another embodiment of a housing 170 illustrated in FIG. 13, port housings 174a, 174b, 174c of sealing ports 172a, 172b, 172c seated substantially flush with a proximal surface 170a of the housing 170 can have non-circular cross-sectional shapes configured as visual and/or tactile identifiers. The port housings 174a, 174b, 174c of the differently-sized sealing ports 172a, 172b, 172c can each have a different cross-sectional shape such that the port housings 174a, 174b, 174c can be visualized and/or felt to uniquely identify each of the sealing ports 172a, 172b, 172c. As mentioned above, the port housings 174a, 174b, 174c can have any cross-sectional shape, but in an exemplary embodiment, the port housings 174a, 174b, 174c can each include a different number of surface features 176a, 176b, 176c, such as a number equal to a size of the sealing ports 172a, 172b, 172c, e.g., ten surface features 176a for the 10 mm diameter sealing port 172a, five surface features 176b for the 5 mm diameter sealing port 172b, three surface features 176c for the 3 mm diameter sealing port 172c. The surface features 176a, 176b, 176c can thus be configured as identifiers that uniquely identify sizes of the sealing ports 172a, 172b, 172c. A visual identifier can also be provided in the form of alpha-numeric markings 178a, 178b, 178c for each of the sealing ports 172a, 172b, 172c.

FIG. 14 illustrates still another embodiment of a housing 180 having a plurality of sealing ports 182a, 182b, 182c seated therein, each of the sealing ports 182a, 182b, 182c including a number of surface features 184a, 184b, 184c equal to a size of the sealing ports 182a, 182b, 182c. A visual identifier can also be provided in the form of alpha-numeric markings 186a, 186b, 186c for each of the sealing ports 182a, 182b, 182c, such as on each of the surface features 184a, 184b, 184c as shown.

FIG. 15 illustrates another embodiment of a housing 190 that includes a number of surface features 194 equal to a size of a sealing port 192 seated in the housing 190. In this embodiment, a port housing of the sealing port 192 on a proximal surface 190a of the housing 190 includes the surface features 194. The surface features 194 can have a variety of configurations, such as in the form of a plurality of concentric, axially aligned, stacked rings in contact with one another to form a pyramid shape through which a surgical instrument can be inserted. The rings can have any shape same or different from any of the other rings, such as each being circular as shown. Each differently-sized sealing port seated in the housing 190 can include a different number of surface features corresponding to a size of the sealing ports, e.g., three concentric rings for a 3 mm port, five concentric rings for a 5 mm port, etc. A visual identifier can also be provided in the form of an alpha-numeric marking 196, such as on one of the surface feature 194 as shown.

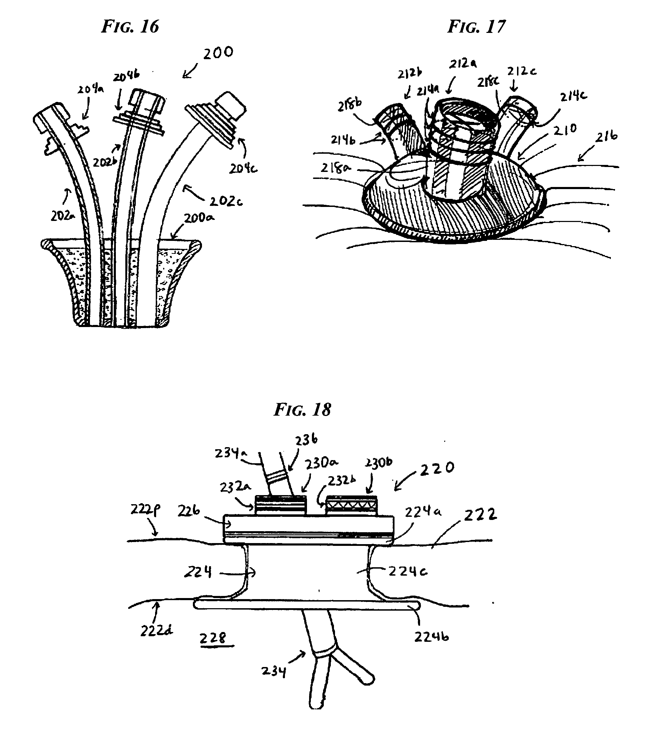

FIG. 16 illustrates another embodiment of a surgical access device 200 including first, second, and third sealing ports 202a, 202b, 202c, each having a plurality of surface features 204a, 204b, 204c positioned above or proximal to a proximal surface 200a of the surgical access device 200. The first and second sealing ports 202a, 202b can each have a 3 mm diameter and three surfaces features 204a, 204b in the form of concentric rings, while the third sealing port 202c can have a 5 mm size and five surface features 204c.

In some embodiments, a housing of a surgical access device can include a number of surface features identifiers that are not equal to sizes of sealing ports they respectively uniquely identify. FIG. 17 illustrates another embodiment of a housing 210 including first, second, and third sealing ports 212a, 212b, 212c, each having a plurality of surface features 214a, 214b, 214c in the form of axially aligned rings positioned above or proximal to a proximal surface 210a of the housing 210 around and/or within tubular housings 218a, 218b, 218c of the sealing ports 212a, 212b, 212c. The rings can be integrally formed with the housings 218a, 218b, 218c or removably mated thereto. The rings can be longitudinally spaced equidistantly or any other distance apart and extend around a perimeter of the sealing ports 212a, 212b, 212c. The rings can have any size, shape, and configuration, same or different from any of the other rings. The sealing ports 212a, 212b, 212c in this illustrated embodiment each have a substantially circular cross-sectional shape, so the rings can have a similar substantially circular shape. Each of the sealing ports 212a, 212b, 212c can have a different size and hence each have a different number of surface features 214a, 214b, 214c, with the largest sealing port 212a having the largest number of surface features 214a, e.g., three, the smallest sealing port 212c having the smallest number of surface features 214c, e.g., one, and the mid-size sealing port 212b having a number of surface features 214b between the largest and smallest, e.g., two. A visual identifier can also be provided in the form of color, such as the port housings 218a, 218b, 218c each having the same color, e.g., transparent, with the rings on each of the housings 218a, 218b, 218c being a different color, e.g., red for the first sealing port 212a, blue for the second sealing port 212b, and yellow for the third sealing port 212c. FIG. 17 also illustrates the housing 210 positioned adjacent tissue 216 with a retractor (not shown) positioned within the tissue 216 to provide access therethrough.

In use, any of the surgical access devices described herein can be positioned within tissue to provide access to a body cavity underlying the tissue. As illustrated in one embodiment in FIG. 18, a surgical access device 220 in use can be positioned within a tissue 222 in a variety of ways. In one embodiment, the device 220 can be positioned in tissue fully assembled in a default position shown in FIG. 18. In another embodiment, the device 220 can be positioned partially assembled in the tissue 222 and be fully assembled with a portion of the device 220 positioned in the tissue 222, e.g., a retractor 224 of the device 220 can first be positioned in the tissue 222 and a housing 226 of the device 220 subsequently coupled to the retractor 224. If the tissue 222 and/or the retractor 224 are adequately flexible, the retractor 224 can be angled or pivoted to a desired position to ease attachment of the housing 226 to the retractor 224.

However positioned within the tissue 222, as illustrated in this embodiment, the retractor 224 as fully assembled can be positioned within an opening or incision formed in the tissue 222, e.g., in the umbilicus, with proximal and distal flanges 224a, 224b of the retractor 224 positioned on opposed sides of the tissue 222. The proximal flange 224a in a proximal portion of the retractor 224 can be positioned on one side of the tissue 222 with a distal surface of the proximal flange 224a positioned on and/or proximal to a proximal surface 222p of the tissue 222. The distal flange 224b of the retractor 224 can be positioned on and/or distal to a distal surface 222d of the tissue 222 in a body cavity 228 underlying the tissue 222. An inner elongate portion 224c of the retractor 224 can thereby be positioned within the tissue 222 with a working channel or passageway (not shown) of the retractor 224 extending through the tissue 222 to provide a path of access to the body cavity 228.

With the surgical access device 220 assembled and positioned in the tissue 222, one or more surgical instruments can be inserted therethrough and into the body cavity 228 where the instruments can help perform any type of surgical procedure. One or more surgical instruments can be inserted through the device 220 and into the body cavity 228 through any of the device's first and second sealing ports 230a, 230b, e.g., a pair of movable jaws 234 inserted through the first sealing port 230a, to help perform at least a portion of a surgical procedure. If the tissue 222 and/or the retractor 224 are adequately flexible, the retractor 224 can be angled or pivoted during use of the device 220 with the movable jaws 234 and/or other surgical tools inserted therethrough. Although a pair of movable jaws 234 are shown inserted through the device 220, any surgical device such as a grasper, a scoping device (e.g., an endoscope, a laparoscope, and a colonoscope), a cutting instrument, etc., can be inserted through the device 220. A person skilled in the art will appreciate that the term "grasper" as used herein is intended to encompass any surgical instrument that is configured to grab and/or attach to tissue and thereby manipulate the tissue, e.g., forceps, retractors, movable jaws, magnets, adhesives, stay sutures, etc. A person skilled in the art will also appreciate that the term "cutting instrument" as used herein is intended to encompass any surgical instrument that is configured to cut tissue, e.g., a scalpel, a harmonic scalpel, a blunt dissector, a cautery tool configured to cut tissue, scissors, an endoscopic linear cutter, a surgical stapler, etc.

The device 220 can include at least one identifier for each of the first and second sealing ports 230a, 230b configured to indicate sizes of the working channels defined by the sealing ports 230a, 230b. In this embodiment, the identifiers include first and second visual identifiers 232a, 232b respectively printed on or otherwise applied as patterns on sidewalls of the first and second sealing ports 230a, 230b, e.g., striped lines for the first sealing port 230a and a zig-zag line for the second sealing port 230b. The surgical instrument 234 can optionally include a visual and/or tactile identifier 236, e.g., striped lines on an elongate shaft 234a of the instrument 234, that can be matched with one of the sealing port identifiers 232a, 232b to facilitate proper insertion of the instrument 234 through the device 220.

At any point before, during, or after a surgical procedure, the housing 226 in full or part can be released from the retractor 224, and the retractor 224 can be removed from the tissue 222. With the housing 226 of the device 220 disengaged from the retractor 224, the passageway of the retractor 224 can still provide access to the body cavity 228 underlying the tissue 222. One or more surgical instruments can be advanced through the passageway of the retractor 224, such as a waste removal bag configured to hold waste material, e.g., dissected tissue, excess fluid, etc., from the body cavity 228. The bag can be introduced into the body cavity 228 through the retractor's passageway or other access port. A person skilled in the art will appreciate that one or more surgical instruments can be advanced through the retractor's passageway before and/or after the housing 226 has been attached to the retractor 224.

As will be appreciated by those skilled in the art, any and all of the embodiments disclosed herein can be interchangeable with one another as needed. For example, an exemplary surgical access device kit could include multiple housings and seal bases with one or more retractors. Each seal base and housing combination can have different quantities and/or sizes of sealing ports enabling various combinations of variously sized surgical instruments to be inserted therethrough as needed in particular application. Various release mechanism known in the art can be used to releasably attach the various base members and housings to a retractor.

There are various features that can optionally be included with any and all of the surgical access device embodiments disclosed herein. For example, a component of the device, such as a seal base, housing, retractor, etc., can have one or more lights formed thereon or around a circumference thereof to enable better visualization when inserted within a patient. As will be appreciated, any wavelength of light can be used for various applications, whether visible or invisible. Any number of ports can also be included on and/or through the surgical access devices to enable the use of various surgical techniques and devices as needed in a particular procedure. For example, openings and ports can allow for the introduction of pressurized gases, vacuum systems, energy sources such as radiofrequency and ultrasound, irrigation, imaging, etc. As will be appreciated by those skilled in the art, any of these techniques and devices can be removably attachable to the surgical access device and can be exchanged and manipulated as needed.

The embodiments described herein can be used in any known and future surgical procedures and methods, as will be appreciated by those skilled in the art. For example, any of the embodiments described herein can be used in performing a sleeve gastrectomy and/or a gastroplasty, as described in U.S. Application No. 12/242,765 entitled "Surgical Access Device" filed on September 30, 2008; U.S. Application No. 12/242,711 entitled "Surgical Access Device with Protective Element" filed on September 30, 2008; U.S. Application No. 12/242,721 entitled "Multiple Port Surgical Access Device" filed on September 30, 2008; U.S. Application No. 12/242,726 entitled "Variable Surgical Access Device" filed on September 30, 2008; U.S. Application No. 12/242,333 entitled "Methods and Devices for Performing Gastrectomies and Gastroplasties" filed on September 30, 2008; U.S. Application No. 12/242,353 entitled "Methods and Devices for Performing Gastrectomies and Gastroplasties" filed on September 30, 2008; and U.S. Application No. 12/242,381 entitled "Methods and Devices for Performing Gastroplasties Using a Multiple Port Access Device" filed on September 30, 2008, all of which are hereby incorporated by reference in their entireties.

The devices disclosed herein can be designed to be disposed of after a single use, or they can be designed to be used multiple times. In either case, however, the device can be reconditioned for reuse after at least one use. Reconditioning can include any combination of the steps of disassembly of the device, followed by cleaning or replacement of particular pieces, and subsequent reassembly. In particular, the device can be disassembled, and any number of the particular pieces or parts of the device can be selectively replaced or removed in any combination, e.g., a seal base, a housing, a proximal retractor base, etc. Upon cleaning and/or replacement of particular parts, the device can be reassembled for subsequent use either at a reconditioning facility, or by a surgical team immediately prior to a surgical procedure. Those skilled in the art will appreciate that reconditioning of a device can utilize a variety of techniques for disassembly, cleaning/replacement, and reassembly. Use of such techniques, and the resulting reconditioned device, are all within the scope of the present application.

Preferably, the invention described herein will be processed before surgery. First, a new or used instrument is obtained and if necessary cleaned. The instrument can then be sterilized. In one sterilization technique, the instrument is placed in a closed and sealed container, such as a plastic or TYVEK bag. The container and instrument are then placed in a field of radiation that can penetrate the container, such as gamma radiation, x-rays, or high-energy electrons. The radiation kills bacteria on the instrument and in the container. The sterilized instrument can then be stored in the sterile container. The sealed container keeps the instrument sterile until it is opened in the medical facility.

It is preferred that device is sterilized. This can be done by any number of ways known to those skilled in the art including beta or gamma radiation, ethylene oxide, steam, and a liquid bath (e.g., cold soak).

One skilled in the art will appreciate further features and advantages of the invention based on the above-described embodiments. Accordingly, the invention is not to be limited by what has been particularly shown and described, except as indicated by the appended claims. All publications and references cited herein are expressly incorporated herein by reference in their entirety.

## Claims

1. A surgical device, comprising:
a housing; and
at least two sealing ports extending through the housing, each sealing port defining a working channel extending therethrough and configured to receive an instrument therethrough, wherein each working channel has a size that differs from a size of another one of the working channels, and wherein each sealing port is associated with at least one of a visual identifier and a tactile identifier configured to indicate the size of the working channel.

2. The device of claim 1, wherein the visual indicator includes at least one of a color, a shape, and a light.

3. The device of claim 1, wherein the visual indicator includes at least one of a numerical marking and an alphabetical marking.

4. The device of claim 1, wherein the visual indicator includes differing vertical heights of the sealing ports.

5. The device of claim 1, wherein the tactile identifier includes a textured surface.

6. The device of claim 1, wherein each sealing port is associated with a visual identifier and a tactile identifier.

7. The device of claim 1, further comprising a retractor configured to attach to the housing and having a working channel extending therethrough for forming a pathway through tissue into a body cavity.

8. A surgical device, comprising:
a housing having first and second sealing ports, each sealing port having a sealing element configured to form a seal around an instrument inserted through the sealing port, the first sealing port having a first diameter and the second sealing port having a second diameter greater than the first diameter;
a first identifier configured to uniquely identify the first sealing port; and
a second identifier configured to uniquely identify the second sealing port.

9. The device of claim 8, wherein at least one of the first and second identifiers is a visual indicator.

10. The device of claim 9, wherein the visual indicator includes at least one of a color, a shape, and a light.

11. The device of claim 9, wherein the visual indicator includes at least one of a numerical marking and an alphabetical marking.

12. The device of claim 8, wherein at least one of the first and second identifiers is a tactile feature, such as a textured surface.

13. The device of claim 8, wherein each of the first and second identifiers has a different cross-sectional shape.

14. The device of claim 8, wherein the first and second identifiers each include a different number of surface features, for example a number of surface features equal to the first and second diameters, respectively.

15. The device of claim 8, further comprising a flexible retractor configured to couple to the housing.
